# FASCICULE DE BREVET EUROPEEN

(11) **EP 2 637 645 B1**
(45) Date de publication et mention de la délivrance du brevet: **21.02.2018**
(21) Numéro de dépôt: 11796739.8
(22) Date de dépôt: 10.11.2011
(51) Int. Cl.: A61K 9/48, A61K 31/343, A61K 31/00, A61K 9/00

(54) **COMPOSITION PHARMACEUTIQUE ET FORME GALENIQUE A BASE DE DRONEDARONE ET SON PROCEDE DE PREPARATION**
PHARMAZEUTISCHE ZUSAMMENSETZUNG UND DARREICHUNGSFORM MIT DRONEDARON UND HERSTELLUNGSVERFAHREN DAFÜR
PHARMACEUTICAL COMPOSITION AND DOSAGE FORM COMPRISING DRONEDARONE, AND PREPARATION METHOD THEREOF

(30) Priorité: 10.11.2010 FR 1059306
(43) Date de publication de la demande: 18.09.2013
(73) Titulaire: SANOFI, 75008 Paris (FR)
(72) Inventeur: ABRAMOVICI, Bernard, F-75013 Paris (FR); BEILLES, Stéphane, F-75013 Paris (FR); GAUTIER, Jean-Claude, F-75013 Paris (FR); CHAMBONNET, Sandra, F-75013 Paris (FR)
(74) Mandataire: Bouron, Estelle
(86) Numéro de dépôt international: PCT/FR2011/052622
(87) Numéro de publication internationale: WO 2012/063005

(56) Documents cités:
- WO-A1-02/45693
- WO-A2-2005/048979
- FR-A1- 2 764 800

## Description

La présente invention se rapporte, d'une manière générale, à une composition pharmaceutique pour administration orale contenant un principe actif à activité antiarythmique. Plus précisément, l'invention concerne une composition pharmaceutique semi-solide ou liquide destinée à être utilisée avantageusement sous forme galénique de type capsule, ladite composition comprenant au moins un dérivé de benzofurane, en tant que principe actif, à activité antiarythmique et au moins un excipient lipidique.

La présente invention concerne également un procédé de préparation d'une telle forme galénique à base de ladite composition pharmaceutique et est aussi relative à l'application en thérapeutique de telle composition ou de telle forme galénique.

Par "dérivé de benzofurane à activité antiarythmique", on désigne dans le cadre de la présente invention, un composé benzofuranique choisi parmi ceux décrits dans les brevets US 3248401, US 5223510 et EP 338746 ainsi que dans les demandes de brevet WO 88/07996, WO 89/02892, WO 90/02743 et WO 94/29289.

De l'ensemble de ces composés, on peut citer, le 2-n-butyl-3-[4-(3-di-n-butylaminopropoxy)benzoyl]-5-méthylsulfonamidobenzo-furane ou dronédarone et ses sels pharmaceutiquement acceptables décrits dans le brevet EP1315709 de même que le 2-n-butyl-3-(3,5-diiodo-4-diéthylaminoéthoxy-benzoyl) benzofurane ou amiodarone et ses sels pharmaceutiquement acceptables décrits dans le brevet US 3248401. WO2005/048979 décrit une composition pharmaceutique à libération modifiée comprenant des micro comprimés comprenant une substance active comme la dronédarone et enrobée d'une couche de contrôle contenant un excipient lipidique amphiphile de valeur HLB 1-20 (p.e.les esters de acide gras, le glycéryle monostéarate; le glycerol monooleate, ou Geleol.

WO0245693 décrit une matrice comprenant une substance active et diffèrent excipients lipidiques amphiphiles de HLB 2-20. Avantageusement, le dérivé de benzofurane à activité antiarythmique est choisi parmi la dronedarone ou 2-n-butyl-3-[4-(3-di-n-butylaminopropoxy)benzoyl]-5-methylsulfonamido-benzofurane de formule (D) sous forme de base libre représentée ci-dessous et ses dérivés, tels que des sels pharmaceutiquement acceptables décrits plus bas.

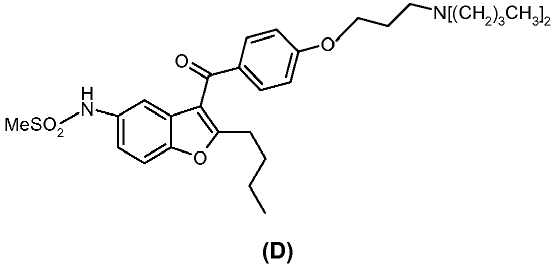

Par "sel Pharmaceutiquement acceptable", on entend un sel qui n'est pas toxique pour l'individu auquel il est administré lorsqu'il est administré à des doses standard. On pourra ainsi nommer comme sels pharmaceutiquement acceptables de la dronédarone, par exemple le chlorhydrate de 2-n-butyl-3-[4-(3-di-n-butylaminopropoxy)benzoyl]-5-methylsulfonamido-benzofurane le fumarate de 2-n-butyl-3-[4-(3-di-n-butylaminopropoxy)benzoyl]-5-methylsulfonamido-benzofurane et l'oxalate de 2-n-butyl-3-[4-(3-di-n-butylaminopropoxy)benzoyl]-5-methylsulfonamido-benzofurane.

Les composés antiarythmiques utilisés dans le cadre de l'invention, notamment la dronédarone et l'amiodarone, sous forme de base, ou leurs sels, en particulier leurs sels de chlorhydrate, sont caractérisés par une faible solubilité en milieu aqueux, ce qui constitue un inconvénient majeur pour une mise à disposition du principe actif par voie orale. Ainsi, la solubilité de ces composés antiarythmiques est faible en milieu gastrique simulé (3 mg/ml à pH = 1,5) et très faible en milieu intestinal simulé (1 µg/ml à pH = 6,5).

A titre d'exemple, la courbe de solubilité du chlorhydrate de dronédarone à température ambiante et en fonction du pH, révèle une solubilité maximale d'environ 1 à 2 mg/ml vers les pH de 3 à 5, mais très faible à des pH de l'ordre de 6 à 7 puisqu'elle n'est plus que de 10 µg/ml à pH = 7. Quant au chlorhydrate d'amiodarone, sa solubilité est, à température ambiante, de 0,3 à 0,9 mg/ml dans la gamme de pH de 3 à 4 et de quelques µg/ml à pH = 7. Ainsi, il est possible de dissoudre 400 mg de chlorhydrate de dronédarone dans 200 ml de milieu aqueux tamponné à pH = 4 (solution aqueuse 0,1M en NaH₂PO₄). Par contre, dans ce milieu dilué au 1/10 par une solution aqueuse tamponnée à pH = 7 (solution aqueuse 0,1M en Na₂HPO₄), le chlorhydrate de dronédarone précipite (pH du milieu final= 6,7). Ces conditions de solubilité étant semblables à celles enregistrées dans le tractus gastro-intestinal, on peut supposer que le chlorhydrate de dronédarone va être soumis dans l'estomac à des conditions acides favorables à sa solubilisation mais dès son entrée dans l'intestin, il va au contraire rencontrer un milieu de pH entre 6 et 7, c'est-à-dire un milieu non solubilisant, dans lequel il risque de précipiter.

Or, c'est principalement au niveau intestinal qu'a lieu l'absorption du principe actif et il est désormais bien connu qu'une administration par voie orale nécessite un maintien en solution optimal du principe actif, afin d'espérer obtenir une perméation suffisante au cours du tractus gastro-intestinal, et donc une exposition acceptable, pour un effet thérapeutique significatif.

Compte tenu du problème de solubilité et de biodisponibilité, il a été mis au point une forme galénique actuellement sur le marché, se présentant sous la forme d'un comprimé pelliculé de 426mg de chlorhydrate de dronédarone correspondant à 400 mg de dronédarone, vendu sous le nom commerciale de Multaq® et dont la posologie recommandée chez l'adulte est la prise d'un comprimé deux fois par jour et ce impérativement pendant les repas pour assurer une action optimale dudit principe actif.

En effet, d'un point de vue pharmacocinétique, après son administration orale lors d'un repas, la dronédarone est bien absorbée (au moins 70%). Toutefois, du fait d'un métabolisme de premier passage présystémique, la biodisponibilité absolue de ce médicament (pris avec de la nourriture) n'est plus que de 15%. La consommation concomitante d'aliments multiplie la biodisponibilité du produit par un facteur 2 à 4 par rapport à la prise du médicament sans prise d'aliments simultanée. Après une administration orale lors d'un repas, les concentrations plasmatiques maximum en dronédarone et en son principal métabolite actif circulant (métabolite N-débutylé) sont atteintes en 3 à 6 heures. La pharmacocinétique de la dronédarone et de son métabolite N-débutylé dévie modérément de la règle de la proportionnalité à la dose: un doublement de la dose entraîne une hausse de la Cmax et de l'AUC par un facteur d'environ 2,5 à 3,0.

Or, il est bien entendu préférable pour un patient de pouvoir bénéficier d'un traitement thérapeutique sans contrainte de prise pendant ou en dehors des repas, tout particulièrement dans le domaine du traitement des troubles du rythme cardiaque, en particulier des arythmies.

La mise au point d'une composition pharmaceutique pour l'administration par voie orale d'un principe actif à activité antiarythmique, capable d'engendrer une biodisponibilité acceptable, indépendamment de l'absorption ou non de nourriture de façon concomitante, c'est-à-dire une composition impliquant un effet repas restreint pour être efficace, reste donc d'un intérêt essentiel.

On a maintenant trouvé de manière tout à fait surprenante et inattendue une nouvelle composition pharmaceutique permettant une administration orale d'au moins un principe actif antiarythmie, avantageusement le 2-n-butyl-3-[4-(3-di-n-butylaminopropoxy)benzoyl]-5-methylsulfonamido-benzofurane ou un dérivé de celui-ci tel que par exemple un de ses sels, sans les inconvénients mentionnés ci-dessus. Cette composition comprenant au moins un principe actif inclus dans une matrice constituée par les autres ingrédients de ladite composition, en particulier les autres excipients, s'avère suffisamment stable et présente une solubilité appropriée pour perdurer dans le tractus gastro-intestinal jusqu'au site d'absorption. Cette composition peut, en outre, être prise à jeun ou avec une collation légère voire un repas à faible apport en graisses et en une ou plusieurs prises.

La présente invention selon revendication 1 a ainsi pour objet une composition pharmaceutique pour l'administration orale d'un principe actif à activité antiarythmique, tel que par exemple le 2-n-butyl-3-[4-(3-di-n-butylaminopropoxy)benzoyl]-5-methylsulfonamido-benzofurane (i) sous forme de base, (ii) sous forme d'un sel pharmaceutiquement acceptable, caractérisée en ce qu'elle comprend outre ledit principe actif, au moins un excipient lipidique amphiphile de valeur HLB comprise entre 5 et 18 en ce que ledit excipient lipidique est choisi parmi les polyoxylglycerides substitués semi-solides. Ladite composition selon l'invention peut se présenter sous une forme galénique de type capsule ou gélule semi-solide ou liquide. En effet, elle peut être avantageusement conditionnée sous une forme galénique de type capsule, encore plus avantageusement de type capsule à enveloppe dure.

Dans le cadre de la présente invention, on entend par :
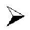 Capsule, une forme galénique à enveloppe dure ou mole ;
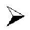 Gélule, une capsule à enveloppe dure, présentant deux parties : une partie appelée corps et une partie appelé tête ;
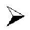 Biodisponibilité, un terme utilisé pour décrire une propriété pharmacocinétique des médicaments, à savoir, la fraction d'une dose qui atteint la circulation sanguine. Elle évalue la quantité de médicament absorbée qui atteint la circulation sanguine et la vitesse d'absorption dudit médicament ;
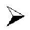 Principe Actif, toute substance possédant un effet thérapeutique, tel que par exemple un effet antiarythmique . Dans le cadre de l'invention, il s'agit en particulier de tout dérivé de benzofurane à activité antiarythmique, défini ci-dessous, en particulier la dronédarone sous forme de base, sous forme de sels pharmaceutiquement acceptables d'addition à des acides organiques ou inorganiques,. Lesdits sels peuvent être préparés avec des acides pharmaceutiquement acceptables, mais les sels d'autres acides utiles, par exemple, pour la purification ou l'isolement des composés de formule (I) font également partie de l'invention.
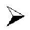 Excipient, toute substance inactive ou inerte vis-à-vis d'un organisme vivant, contrairement au principe actif et qui facilite la préparation et l'administration d'un médicament ;
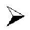 Excipient lipidique, tout excipient connu de l'homme du métier comme solvant lipidique, avantageusement amphiphile, présentant une valeur HLB (terme défini ci-après), qui selon l'invention est inférieur à 20 et supérieur à 1;
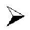 Matrice, l'ensemble des ingrédients autres que le ou les principes actifs de la composition selon l'invention, en particulier les excipients ;
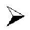 Valeur HLB, la valeur balance hydrophile-lipophile, selon la classification mise au point par Griffin, bien connue de l'homme du métier ;
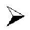 Tensioactif, un excipient, qui de par ses propriétés amphiphiles, facilite le mouillage des poudres, améliore la solubilité/solubilisation et/ou ralentit la reprécipitation;
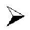 Co-solvant, tout solvant améliorant la faisabilité du procédé de fabrication de la composition selon l'invention sur la base des paramètres clés de viscosité et de point de fusion de la matrice de ladite composition ainsi que de la solubilisation ou de la dispersion du principe actif dans ladite matrice;
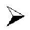 Diluant, un excipient servant à obtenir un volume de composition suffisant pour fabriquer une forme galénique, par exemple une gélule, de la taille désirée et possédant les caractéristiques physiques adaptées à la technique de fabrication choisie pour la gélule;
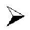 Désintégrant, un excipient qui permet le délitement satisfaisant, de la forme galénique et donc la désintégration du principe actif dans l'estomac en augmentant la friabilité et en diminuant la dureté de la forme galénique;
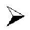 Antiadhérent, un excipient destiné à empêcher que les particules ne collent entre elles et au matériel de fabrication au moment de la fabrication de la forme galénique, par exemple au moment du remplissage des gélules.
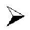 Lubrifiant, un excipient destiné à faciliter les étapes de fabrication des formes galéniques, grâce à leur rôle glissant, c'est à dire consistant à augmenter la fluidité des particules dans les tubulures des machines ;
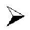 Plastifiant, un excipient destiné à permettre une libération constante du principe actif de la forme galénique en s'intercalant entre les chaînes de polymères et en leur permettant de glisser les unes par rapport aux autres. Il est choisi en fonction de sa solubilité.

Parmi les compositions selon l'invention, on peut citer un premier groupe de compositions pharmaceutiques comprenant de :
- 1-60% en poids d'au moins un principe actif conforme à l'invention, avantageusement entre 1 et 50 %, encore plus avantageusement entre 10 et 45%, mieux encore entre 20% et 40% ;
- 40-99% en poids d'au moins un excipient lipidique conforme à l'invention, avantageusement entre 45 et 80%, encore plus avantageusement entre 50% et 60%,
- 0-30% en poids d'au moins un composé choisi parmi les tensio-actifs, les co-solvants, les diluants, les désintégrants, les lubrifiants, les bases organiques ou inorganiques et les plastifiants, avantageusement de 1 à 20%, mieux encore de 1 à 10%,
les % étant exprimés en poids par rapport au poids total de ladite composition.

Parmi les compositions selon l'invention, on peut citer un deuxième groupe de compositions pharmaceutiques comprenant de :
- 1-60% en poids d'au moins un principe actif conforme à l'invention, avantageusement entre 1 et 50 %, encore plus avantageusement entre 10 et 45%, mieux encore entre 20% et 40% ;
- 37-99% en poids d'au moins un excipient lipidique conforme à l'invention, avantageusement entre 45 et 80%, encore plus avantageusement entre 45% et 55%,
- 0-30% en poids d'au moins un composé choisi parmi les tensio-actifs, les co-solvants, les diluants, les désintégrants, les lubrifiants, les bases organiques ou inorganiques et les plastifiants, avantageusement de 1 à 20%, mieux encore de 1 à 10%,
les % étant exprimés en poids par rapport au poids total de ladite composition.

Parmi les compositions selon l'invention, on peut citer un troisième groupe de compositions pharmaceutiques comprenant de :
- 1-60% en poids d'au moins un principe actif conforme à l'invention, avantageusement entre 1 et 50 %, encore plus avantageusement entre 10 et 45%, mieux encore entre 20% et 40% ;
- 40-99% en poids d'au moins un excipient lipidique conforme à l'invention, avantageusement entre 45 et 80%, encore plus avantageusement entre 50% et 60%,
- 0-30% en poids d'au moins un tensio-actif, avantageusement entre 1% à 20%, encore plus avantageusement entre 5% à 15%, et
- 0-29% en poids d'au moins un co-solvant, avantageusement entre 1 et 20%, encore plus avantageusement entre 2 et 15% ;
les % étant exprimés en poids par rapport au poids total de ladite composition. Le total des compositions fait 100% en poids.

Parmi les compositions selon l'invention, on peut citer un quatrième groupe de compositions pharmaceutiques comprenant de :
- 1-60% en poids d'au moins un principe actif conforme à l'invention, avantageusement entre 1 et 50 %, encore plus avantageusement entre 10 et 45%, mieux encore entre 20% et 40% ;
- 37-99% en poids d'au moins un excipient lipidique conforme à l'invention, avantageusement entre 45 et 80%, encore plus avantageusement entre 50% et 60%,
- 0-30% en poids d'au moins un tensio-actif, avantageusement entre 1% à 20%, encore plus avantageusement entre 5% à 10%, et
- 0-29% en poids d'au moins un co-solvant, avantageusement entre 1 et 20%, encore plus avantageusement entre 2 et 15% ;
les % étant exprimés en poids par rapport au poids total de ladite composition. Le total des compositions fait 100% en poids.

Parmi les compositions selon l'invention, on peut citer un cinquième groupe de compositions pharmaceutiques comprenant de :
- 60-200% en poids d'au moins un excipient lipidique conforme à l'invention, avantageusement entre 120 et 180%, encore plus avantageusement 180%
- 0-30% en poids d'au moins un tensio-actif, avantageusement entre 5% à 30%, encore plus avantageusement entre 10% à 30%,
- 0-30% en poids d'au moins un co-solvant, avantageusement entre 1 et 20% ;
les % étant exprimés en poids par rapport au poids total principe actif.

Les compositions pharmaceutiques selon l'invention comprennent au moins un principe actif à activité antiarythmique et au moins un excipient lipidique.

Parmi les principes actifs à activité antiarythmique conformes à l'invention, on peut citer le 2-n-butyl-3-[4-(3-di-n-butylaminopropoxy)benzoyl]-5-méthylsulfonamidobenzofurane sous forme de base ou dronédarone et ses sels pharmaceutiquement acceptables décrits dans le brevet EP1315709.

Comme sels pharmaceutiquement acceptable, on peut citer par exemple le chlorhydrate de 2-n-butyl-3-[4-(3-di-n-butylaminopropoxy)benzoyl]-5-méthylsulfonamidobenzofurane, le fumarate de 2-n-butyl-3-[4-(3-di-n-butylaminopropoxy)benzoyl]-5-méthylsulfonamidobenzofurane et l'oxalate de 2-n-butyl-3-[4-(3-di-n-butylaminopropoxy)benzoyl]-5-méthylsulfonamidobenzofurane.

Avantageusement, la composition selon l'invention comprend le 2-n-butyl-3-[4-(3-di-n-butylaminopropoxy)benzoyl]-5-méthylsulfonamidobenzofurane ou le chlorhydrate de 2-n-butyl-3-[4-(3-di-n-butylaminopropoxy)benzoyl]-5-méthylsulfonamidobenzofurane en tant que principe actif.

Selon une variante, la solubilisation du principe actif sous sa forme base peut être obtenue en partant de sels pharmaceutiquement acceptables de dronédarone, tels que mentionnés ci-dessus, et en effectuant la reformation du principe actif sous sa forme base in situ par changement de pH avec une base organique ou inorganique.

Les compositions selon l'invention comprenant au moins un sel pharmaceutiquement acceptable de dronédarone, et comprenant en outre au moins une base organique ou inorganique, avantageusement en quantité stoechiométrique molaire par rapport au principe actif sous forme de base, font partie de la présente invention.

A titre indicatif, la nature de la base pouvant être utilisée dans la composition peut être organique, telle que par exemple l'éthanolamine, ou minérale telle que par exemple la soude ou la potasse. Avantageusement, il s'agit de la soude.

Le ou les principe(s) actif(s) conforme(s) à l'invention est ou sont présent(s) dans la composition selon l'invention dans une proportion allant de 1-60% en poids, avantageusement entre 1 et 50 %, encore plus avantageusement entre 10 et 45%, mieux encore entre 20% et 40% en poids par rapport au poids total de la composition.

L'excipient lipidique est un excipient lipidique amphiphile de valeur HLB comprise entre 1 et 20 et dont la température de fusion est inférieure à 50°C.

L'excipient lipidique est un excipient lipidique amphiphile de valeur HLB comprise entre 2 et 20 et dont la température de fusion est inférieure à 50°C.

On distingue les excipients amphiphiles semi-solides à température ambiante et les excipients amphiphiles liquides à température ambiante.

L'excipient lipidique conforme à l'invention peut être choisi parmi :
- Les glycérides substitués semi-solides,
- Les glycérides substitués liquides,
- Les polyoxylglycerides substitués semi-solides,
- Les polyoxylglycerides substitués liquides,
- Et leurs mélanges.

On peut, par exemple, citer un groupe dans lequel l'excipient lipidique est choisi parmi :
- comme glycérides substitués semi-solides conformes à l'invention, les gélucires commercialisés par exemple sous la marque Gelucire® 33/01, Gelucire® 39/01 Gelucire® 43/01 et Geleol®, Peceol™,
- comme glycérides substitués liquides conformes à l'invention, ceux commercialisés par exemple sous le nom de Labrafac Lipophile® WL1349,
- comme polyoxylglycerides substitués semi-solides conformes à l'invention, les Gélucire commercialisés par exemple sous la marque Gélucire®44/14, Gélucire ®50/13,
- comme polyoxylglycerides substitués liquides conformes à l'invention, ceux commercialisés par exemple sous la marque Labrafil®M1944CS, Labrafil®M2125CS, Labrafil®M2130CS et Labrasol®,

On peut, par exemple, citer un autre groupe dans lequel l'excipient lipidique est choisi parmi des polyoxylglycerides substitués semi-solides conformes à l'invention, les Gélucire commercialisés plus particulièrement le lauroyl macroglyceride commercialisé sous la marque Gélucire®44/14.

Lorsque le principe actif se présente sous la forme d'un sel, la composition peut se présenter sous la forme d'une dispersion dudit principe actif solide dans une matrice solide à température ambiante dans le cas où l'on utilise un excipient lipidique ou semi-solide en quantité suffisante ou sous la forme d'une dispersion de solide dans une huile à température ambiante dans le cas où l'on utilise un excipient lipidique liquide en quantité suffisante, la solubilité dudit principe actif dans la composition étant en outre fonction du pH du milieu dans lequel la composition se trouve.

L'excipient lipidique, amphiphile semi-solide à température ambiante, de la composition selon l'invention, a l'avantage de permettre une dispersion solide ou une solubilisation à chaud du principe actif dans la matrice de ladite composition et de faciliter la mise en solution du principe actif lors de la dissolution de la matrice dans le milieu aqueux gastrique et/ou intestinal.

L'excipient lipidique, amphiphile, liquide à température ambiante, de la composition selon l'invention, a l'avantage de faciliter la mise en solution du principe actif dans le milieu aqueux gastrique et/ou intestinal.

Avantageusement, la composition selon l'invention comprend au moins un excipient lipidique, amphiphile, ayant une valeur de HLB comprise entre 5 et 18.

Les excipients lipidiques, amphiphiles, de valeur HLB comprise entre 5 et 18, conformes à l'invention, peuvent être choisis dans le groupe comprenant :
- les mono- et di-glycérides à chaînes moyennes, tel que par exemple le Capmul MCM®(valeur HLB entre 5,5 et 6), commercialisé par la société Abitec,
- le propylène glycol monolaurate, tel que par exemple le Lauroglycol® 90 (valeur HLB égale à 5) et le Capmul PG12®, respectivement commercialisés par les sociétés Gattefossé et Abitec,
- les caprylocaproyl macrogol-8 glycérides, tel que par exemple le Labrasol® (valeur HLB égale à 14), commercialisé par la société Gattefossé,
- les lauroyl macrogolglycérides, tel que par exemple le Gelucire® 44/14 (valeur HLB égale à 14) et le Gelucire® 50/13 (valeur HLB égale à 13), commercialisés par la société Gattefossé,
- le propylène glycol caprylique acide mono ester, tel que par exemple le Capmul® PG-8 (valeur HLB égale à 6), commercialisé par la société Abitec,
- et les mélanges de ceux-ci.

Plus particulièrement, l'excipient lipidique, amphiphile, de valeur HLB comprise entre 5 et 18 est choisi dans le groupe comprenant le Capmul MCM®, le Lauroglycol® 90, le Capmul PG12®, le Labrasol® , le Gelucire® 44/14, le Gelucire® 50/13, le Capmul® PG-8, et les mélanges de ceux-ci.

Selon un mode de réalisation, les excipients lipidiques conformes à l'invention sont choisis parmi les excipients lipidiques, amphiphiles, ayant une valeur de HLB comprise entre 12 et 18.

Le ou les excipients lipidique(s) conforme(s) à l'invention est ou sont présent(s) dans la composition selon l'invention dans une proportion allant de 40-99% en poids, avantageusement entre 45 et 80%, encore plus avantageusement entre 50% et 60% en poids par rapport au poids total de la composition.

Le ou les excipients lipidique(s) conforme(s) à l'invention est ou sont présent(s) dans la composition selon l'invention dans une proportion allant de 37-99% en poids, avantageusement entre 45 et 80%, encore plus avantageusement entre 50% et 60% en poids par rapport au poids total de la composition.

Le ou les excipients lipidique(s) conforme(s) à l'invention est ou sont présent(s) dans la composition selon l'invention dans une proportion allant de 100-200% en poids, avantageusement entre 110 et 180%, encore plus avantageusement entre 50% et 60% en poids par rapport au poids total principe actif.

Les compositions pharmaceutiques selon l'invention peuvent comprendre en outre au moins un tensio-actif et/ou au moins un co-solvant.

L'agent tensioactif est avantageusement hydrophile et/ou non ionique. Il peut être choisi parmi:
- des copolymères oxyde d'éthylène/oxyde de propylène ci-après dénommés poloxamers, tels que le poloxamer 124 commercialisé sous la marque SYNPERONIC PE/L44; le poloxamer 188 commercialisé sous la marque PLURONIC F68 ou SYNPERONIC PE/F68; le poloxamer 237 commercialisé sous la marque PLURONIC F87 ou SYNPERONIC PE/F87; le poloxamer 338 commercialisé sous la marque SYNPERONIC PE/F108 ou le poloxamer 407 commercialisé sous la marque PLURONIC F127, SYNPERONIC PE/F127 ou LUTROL F127 ;
- des huiles de ricin polyéthoxylées, telles que celles commercialisées sous la marque CREMOPHOR RH40 ;
- des polysorbates éthoxylés, tels que les polysorbate 20, polysorbate 40, polysorbate 60 et polysorbate 80 commercialisés respectivement sous les marques TWEEN 20, TWEEN 40, TWEEN 60, et TWEEN 80 ; et
- des hydroxystéarates de polyéthylène, tels que l'hydroxystéarate de polyéthylène 660 commercialisé sous la marque SOLUTOL HS15.

Plus particulièrement, l'agent tensioactif peut être choisi parmi:
- des copolymères oxyde d'éthylène/oxyde de propylène ci-après dénommés poloxamers, tels que le poloxamer 124 commercialisé sous la marque SYNPERONIC PE/L44; le poloxamer 188 commercialisé sous la marque PLURONIC F68 ou SYNPERONIC PE/F68; ou le poloxamer 407 commercialisé sous la marque PLURONIC F127, SYNPERONIC PE/F127 ou LUTROL F127 ;
- des huiles de ricin polyéthoxylées, telles que celles commercialisées sous la marque CREMOPHOR RH40 ;

- des polysorbates éthoxylés, tels que le polysorbate 60 commercialisé sous la marque TWEEN 60; et
- des hydroxystéarates de polyéthylène, tels que l'hydroxystéarate de polyéthylène 660 commercialisé sous la marque SOLUTOL HS15.

Avantageusement, le ou les tensioactifs conforme(s) à l'invention est ou sont choisi(s) parmi les copolymères oxyde d'éthylène/oxyde de propylène dénommés poloxamers, encore plus avantageusement il s'agit du poloxamer 407.

Ledit tensioactif peut être présent dans la composition selon l'invention à raison de 0% à 30% en poids par rapport au poids total de ladite composition, avantageusement entre 1% et 20% en poids, encore plus avantageusement de 5% à 15% en poids de tensio-actif.

Ledit tensioactif peut être présent dans la composition selon l'invention à raison de 0-30% en poids d'au moins un tensio-actif, avantageusement entre 5% à 20%, encore plus avantageusement entre 10% à 20%, en poids par rapport au poids total de principe actif.

L'agent co-solvant conforme à l'invention peut être choisi parmi les solvants organiques alcooliques ou les dérivés de glycol.

On peut citer en tant que co-solvant :
- Les alcools tels que par exemple l'éthanol et l'isopropanol ;
- Le propylène glycol et ses dérivés, éventuellement substitués, tels que ceux commercialisés sous la marque Labrafac® PG, Lauroglycol™ 90, Lauroglycol™FCC, Capryol™90, Capryo™PGMC.

Ledit co-solvant peut être présent dans la composition pharmaceutique selon l'invention à raison de 0% à 29% en poids par rapport au poids total de ladite composition, avantageusement entre 1% et 20% en poids, encore plus avantageusement de 2% à 15% en poids de co-solvant.

Ledit co-solvant peut être présent dans la composition pharmaceutique selon l'invention à raison de 0-30% en poids d'au moins un co-solvant, avantageusement entre 1 et 20%, en poids par rapport au poids total principe actif.

Selon un mode de réalisation, le co-solvant est un dérivé de glycol substitué et/ou à une teneur inférieure à 29% en poids par rapport au poids totale de ladite composition, avantageusement le co-solvant est le Propylène Glycol et/ou à une teneur d'environ 20% en poids.

Selon un mode de réalisation, le co-solvant est un dérivé de glycol substitué et/ou à une teneur inférieure à 30% en poids par rapport au poids total de principe actif, avantageusement le co-solvant est le Propylène Glycol et/ou à une teneur d'environ 20% en poids par rapport au poids total principe actif.

Selon un mode de réalisation, la composition selon l'invention comprend :
- le chlorhydrate de 2-n-butyl-3-[4-(3-di-n-butylaminopropoxy)benzoyl]-5-méthylsulfonamidobenzofurane ou la 2-n-butyl-3-[4-(3-di-n-butylaminopropoxy)benzoyl]-5-méthylsulfonamidobenzofurane sous forme de base, comme principe actif,
   et/ou
- au moins un excipient lipidique semi-solide de HLB entre 1-20, avantageusement entre 5-18, encore plus avantageusement entre 12-18, avantageusement choisi parmi les glycérides substitués semi-solides et les polyoxylglycerides substitués semi-solides, avantageusement il s'agit du Gélucire®44/14,
   et/ou
- au moins un tensioactif, avantageusement choisi parmi les copolymères oxyde d'éthylène/oxyde de propylène dénommé poloxamère, encore plus avantageusement il s'agit du poloxamère 407,
et éventuellement au moins un co-solvant tel que défini ci-dessus.

Cette composition pharmaceutique se présente sous une forme liquide ou semi-solide, c'est-à-dire de consistance pâteuse, en fonction de la consistance et la nature du ou des excipients utilisé(s), entre autre de l'excipient lipidique, à température ambiante. Un excipient lipidique ou semi-solide à température ambiante donnera lieu à la formation d'une matrice semi-solide et donc à une composition selon l'invention de consistance pâteuse alors qu'un excipient lipidique liquide à température ambiante donnera lieu à la formation d'une matrice liquide et donc à une composition selon l'invention de consistance liquide.

Ainsi, dans le cas où l'excipient lipidique conforme à l'invention est choisi parmi les excipients lipidiques ou semi-solides, la composition selon l'invention peut être préparée par la mise en oeuvre de procédés connus de solubilisation ou de dispersion solide à froid ou à chaud dans l'excipient lipidique formant une matrice lipidique. La fabrication de la composition consiste, par exemple, en une solubilisation ou une dispersion du principe actif conforme à l'invention et éventuellement d'autres excipients conformes à l'invention, dans ledit excipient lipidique à une température d'environ 30°C à 60°C, telle que par exemple une température d'environ 44°C, ladite température étant choisie en fonction de la température de fusion dudit excipient lipidique utilisé.

Selon un mode de réalisation particulièrement avantageux, le procédé de fabrication de la composition selon l'invention consiste à solubiliser le principe actif, avantageusement le 2-n-butyl-3-[4-(3-di-n-butylaminopropoxy)benzoyl]-5-méthylsulfonamidobenzofurane sous forme de base, à environ 44°C dans l'excipient lipidique, avantageusement le lauryl macroglycérides, tel que par exemple le Gelucire®44/14.

Dans le cas où l'excipient lipidique, conforme à l'invention, est choisi parmi les excipients lipidiques liquides, la composition selon l'invention peut être préparée par la mise en oeuvre de procédés connus de solubilisation ou de dispersion du principe actif dans l'excipient lipidique formant une matrice lipidique liquide à température ambiante.

Les procédés de préparation des compositions pharmaceutiques selon l'invention se font par les techniques classiques connues de l'homme du métier.

La composition pharmaceutique liquides ou semi-solides, selon l'invention, ainsi obtenue, pourra être ensuite incorporée dans une gélule, telle quelle. L'encapsulation est réalisée selon les procédés classiques d'encapsulation en tenant compte des contraintes physico-chimiques de ladite composition et dudit procédé.

Ladite composition peut éventuellement être transformée en poudres, granulés ou éventuellement susceptibles d'être incorporés dans des gélules ou utilisés tels quels.

L'invention vise ainsi également une forme galénique comprenant une composition pharmaceutique selon l'invention.

Cette forme galénique peut se présenter sous forme de capsule contenant la composition selon l'invention ou éventuellement sous forme de poudres, granulés pouvant être délivrés en récipients multidoses ou sous forme de doses unitaires tels que paquets, sachets.

Les capsules sont des préparations solides constituées d'une enveloppe dure ou molle, de forme et de capacité variables, contenant généralement une dose unitaire de principe actif. L'enveloppe est à base de gélatine ou d'autres substances naturelles ou synthetiques dont la consistance peut être adaptée par addition par exemple de glycérol ou de sorbitol. D'autres excipients tels que des agents tensio-actifs, des opacifiants, des conservateurs antimicrobiens, des édulcorants, des colorants et/ou des aromatisants peuvent également être ajoutés dans la composition des enveloppes de capsules.

On peut citer comme capsules : les gélules, les capsules à enveloppe molle, les capsules gastrorésistantes et les capsules à libération modifiée.

Avantageusement, la forme galénique selon l'invention est une gélule.

Le procédé de fabrication de gélules comportant un corps et une tête, consiste à (i) préparer la composition selon l'invention par mélange des ingrédients tel que défini plus haut puis à (ii) remplir les parties tête et/ou corps de gélule par répartition volumétrique selon un procédé adapté aux poudres (compresso-doseur, un procédé d'arasage, un procédé d'arasage et tassement ou bourrage alternés, un procédé de vis sans fin ou un procédé de dosage en alvéole) ou au semi-solides (coulage du produit fondu ou liquide),et enfin à fermer les gélules par emboitement des parties formant la tête et le corps de ladite gélule.

Dans le cas des Capsules Molles la préparation liquide est coulée en même temps que la capsule est formée dans les matrices selon le procédé classique de fabrication.

A titre indicatif mais non limitatif, la quantité de principe actif peut varier de 50 à 500 mg par unité d'administration telle que par exemple (i) une capsule, avantageusement une gélule, ou (ii) un sachet de poudres, ou granulés, et la quantité d'excipient lipidique entre 0,5 et 100 mg. A titre préférentiel, une forme galénique selon l'invention, par exemple une gélule, peut comprendre de 200 à 400 mg de principe actif.

La composition pharmaceutique selon l'invention et la forme galénique comprenant une telle composition vise à limiter l'effet repas après administration orale chez l'homme. L'excipient lipidique permet d'une part de solubiliser le principe actif conforme à l'invention et d'autre part de le préserver des effets négatifs du pH au niveau du tractus intestinal, permettant ainsi de s'exonérer de l'effet repas de façon significative. La présence d'un tensio-actif, tel que par exemple le poloxamer, dans ladite composition, permet de limiter la reprécipitation et agglomération du principe actif lors du tractus gastro-intestinal.
La figure 1 représente les courbes de dissolution de gélules selon l'invention (Gélules G1 à Gélules G6), d'une gélule de référence comparative et d'un comprimé à 10% de poloxamère comparatif, toutes ces formulations contenant du principe actif conforme à l'invention.
La figure 2 représente les courbes de dissolution de gélules selon l'invention (Gélules G4 à Gélules G8), toutes ces formulations contenant du principe actif conforme à l'invention.
La figure 3 représente les courbes de dissolution de gélules selon l'invention (Gélules G1, G3, G9), toutes ces formulations contenant du principe actif conforme à l'invention.
La figure 4 représente les courbes de dissolution de gélules selon l'invention (Gélules G5, G10, G13), toutes ces formulations contenant du principe actif conforme à l'invention.
La figure 5 représente les courbes de dissolution de gélules selon l'invention (Gélules G1, G9, G11, G12), toutes ces formulations contenant du principe actif conforme à l'invention.
La figure 6 représente les courbes de dissolution de gélules selon l'invention (Gélules G1, G22, G24, G26, G28), toutes ces formulations contenant du principe actif conforme à l'invention.
La figure 7 représente les courbes de dissolution de gélules selon l'invention (Gélules G5, G23, G25, G27, G29), toutes ces formulations contenant du principe actif conforme à l'invention.

Ces courbes expriment le % en poids de principe actif libéré en fonction du temps, exprimé en minutes. La ligne verticale en gras à 60 min matérialise le moment auquel une solution alcaline de NaOH est ajoutée au milieu gastrique simulé afin de simuler un milieu intestinal.

### EXEMPLES

Le tableau A ci-dessous montre la solubilité du chlorhydrate de dronedarone et de la dronedarone forme base dans des excipients lipidiques selon l'invention.

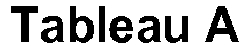

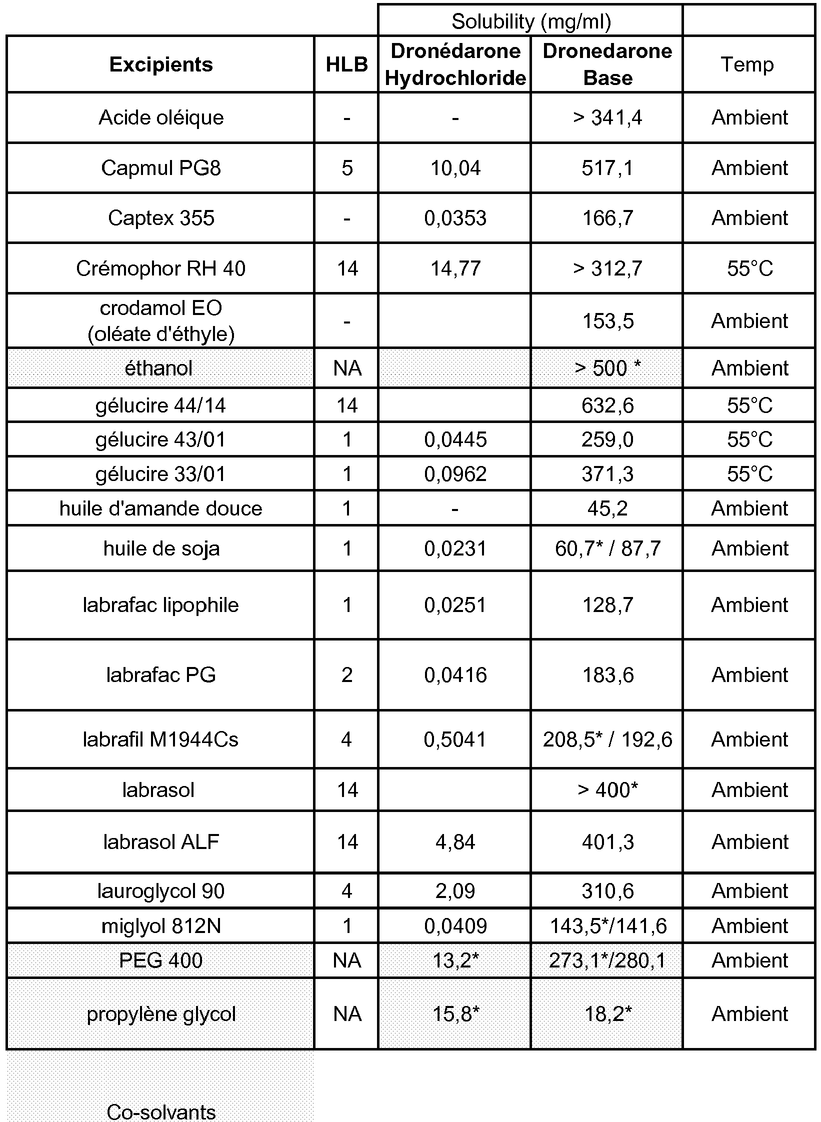

Des compositions selon l'invention ont été fabriquées, dont la composition est détaillée dans les tableaux 1, 3 et 4 ci-dessous.

Des compositions comparatives, non conformes à l'invention, ont été fabriquées dont la composition est détaillée dans le **tableau 2** ci-dessous. Les quantités de composés utilisés pour fabriquer lesdites compositions sont exprimées en mg dans lesdits tableaux 1 et 2.
« - » signifie absent de la composition.
« QS » signifie en quantité suffisante.
« Sel HCl » signifie chlohydrate de dronedarone
« Base » signifie dronedarone forme base.
« Pol. » signifie « poloxamère ».
« Géluc. » signifie « Gélucire ».
« Crém. RH40 signifie « Crémophor RH40) »

**Tableau 1**

| **Ingrédients** | **EX 1 (mg)** | **EX 2 (mg)** | **EX 3 (mg)** | **EX 4 (mg)** | **EX 5 (mg)** | **EX 6 (mg)** |
|---|---|---|---|---|---|---|
| Dronedarone sous forme de chlorhydrate | 213,0 | 213,0 | 213,0 | - | - | - |
| Dronedarone sous forme de base | - | - | - | 200,0 | 200,0 | 200,0 |
| Lauroyl macrogolglycerides (gélucire 44/14) | 357,0 | 343,7 | 237,0 | 343,7 | 357 ,0 | 237,0 |
| Poloxamer 407 | 60,0 | 60,0 | - | 60,0 | 60,0 | - |
| Hydroxyde de sodium* | - | 14,4 | - | - | - | - |
| Eau distillée* | - | 38,9 | - | 38,9 | - | - |
| Propylène glycol | 40,0 | | - | - | 40,0 | - |
| Poids total (mg) | 670,0 | 670,0 | 450,0 | 642,6 | 657,0 | 437,0 |
| **Forme galénique Gélule** | **G1** | **G2** | **G3** | **G4** | **G5** | **G6** |

| | | | | | | |
|---|---|---|---|---|---|---|
| * correspond à une solution aqueuse à 27% d'hydroxyde de sodium | | | | | | |

La composition centésimale de ces formulations G1 à G6 et d'autres formulations selon l'invention est exprimée dans les tableaux 3 et 4 ci-dessous.

**Tableau 3**

| Composition centésimale par rapport à la formule totale | | | | |
|---|---|---|---|---|
| Gélule | % principe actif (éq base) | % de gélucire | % de tensioactif (TA) | % de propylène glycol (co-solvant) |
| G1 | 30 (sel HCl) | 53 (Géluc.44/14) | 9 (Pol. 407) | 6 |
| G2 | 30 (sel HCl) | 51 (Géluc.44/14) | 9 (Pol.407) | 0 |
| G3 | 44 (sel HCl) | 53 (Géluc.44/14) | 0 | 0 |
| G4 | 31 (Base) | 53 (Géluc.44/14) | 9 (Pol.407) | 0 |
| G5 | 30 (Base) | 54 (Géluc.44/14) | 9 (Pol.407) | 6 |
| G6 | 46 (Base) | 54 (Géluc.44/14) | 0 | 0 |
| G7 | 63 (400mg base) | 37 (Géluc.44/14) | 0 | 0 |
| G8 | 36 (200mg base) | 64 (Géluc.44/14) | 0 | 0 |
| G9 (= G1 sans TA) | 33 (sel HCl) | 59 (Géluc.44/14) | 0 | 7 |
| G10 (= G5 sans TA) | 34 (Base) | 60 (Géluc.44/14) | 0 | 7 |
| G11 (= G1 avec 5% TA) | 32 (sel HCl) | 58 (Géluc.44/14) | 2 (Pol.407) | 6 |
| G12 (= G1 avec 10% TA) | 32 (sel HCl) | 57 (Géluc.44/14) | 3 (Pol.407) | 6 |
| G13 (= G5 avec 10% TA) | 32 (Base) | 58 (Géluc.44/14) | 3 (Pol.407) | 6 |
| G14 | 30 (sel HCl) | 53 (Géluc.33/01) | 9 | 6 |
| G15 | 30 (base) | 54 (Géluc.33/01) | 9 | 6 |
| G16 | 33 | 59 | 0 | 7 |
| | (sel HCl) | (Géluc.33/01) | | |
| G17 | 34 (base) | 60 (Géluc.33/01) | 0 | 7 |
| G18 | 30 (sel HCl) | 53 (Géluc.43/01) | 9 | 6 |
| G19 | 30 (base) | 54 (Géluc.43/01) | 9 | 6 |
| G20 | 33 (sel HCl) | 59 (Géluc.43/01) | 0 | 7 |
| G21 | 34 (base) | 60 (Géluc.43/01) | 0 | 7 |
| G22 | 30 (sel HCl) | 53 (Géluc.44/14) | 9 (Pol.188) | 6 |
| G23 | 30 (base) | 54 (Géluc.44/14) | 9 (Pol.188) | 6 |
| G24 | 30 (sel HCl) | 53 (Géluc.44/14) | 9 (Crém. RH40) | 6 |
| G25 | 30 (base) | 54 (Géluc.44/14) | 9 (Crém. RH40) | 6 |
| G26 | 30 (sel HCl) | 53 (Géluc.44/14) | 9 (pluronic L44) | 6 |
| G27 | 30 (base) | 54 (Géluc.44/14) | 9 (pluronic L44) | 6 |
| G28 | 30 (sel HCl) | 54 (Géluc.44/14) | 9 (tween 60) | 6 |
| G29 | 30 (base) | 54 (Géluc.44/14) | 9 (tween 60) | 6 |

**Tableau 4**

| Composition centésimale par rapport au principe actif | | | |
|---|---|---|---|
| Gélule | % de gélucire | % de tensioactif | % de propylène glycol (co-solvant) |
| G1 | 179 (Géluc.44/14) | 30 (Pol.407) | 20 |
| G2 | 172 (Géluc.44/14) | 30 (Pol.407) | 0 |
| G3 | 119 (Géluc.44/14) | 0 | 0 |
| G4 | 172 (Géluc.44/14) | 30 (Pol.407) | 0 |
| G5 | 179 (Géluc.44/14) | 30 (Pol.407) | 20 |
| G6 | 119 (Géluc.44/14) | 0 | 0 |
| G7 | 59 (Géluc.44/14) | 0 | 0 |
| G8 | 179 (Géluc.44/14) | 0 | 0 |
| G9 (= G1 sans TA) | 179 (Géluc.44/14) | 0 | 20 |
| G10 (= G5 sans TA) | 179 (Géluc.44/14) | 0 | 20 |
| G11 (= G1 avec 5% TA) | 179 (Géluc.44/14) | 5 (Pol.407) | 20 |
| G12 (= G1 avec 10% TA) | 179 (Géluc.44/14) | 10 (Pol.407) | 20 |
| G13 (= G5 avec 10% TA) | 179 (Géluc.44/14) | 10 (Pol.407) | 20 |
| G14 | 179 (Géluc. 33/01) | 30 (Pol.407) | 20 |
| G15 | 179 (Géluc. 33/01) | 30 (Pol.407) | 20 |
| G16 | 179 (Géluc. 33/01) | 0 | 20 |
| G17 | 179 (Géluc. 33/01) | 0 | 20 |
| G18 | 179 (Géluc. 43/01) | 30 (Pol.407) | 20 |
| G19 | 179 | 30 | 20 |
| | (Géluc. 43/01) | (Pol.407) | |
| G20 | 179 (Géluc. 43/01) | 0 | 20 |
| G21 | 179 (Géluc. 43/01) | 0 | 20 |
| G22 | 179 (Géluc.44/14) | 30 (Pol. 188) | 20 |
| G23 | 179 (Géluc.44/14) | 30 (Pol. 188) | 20 |
| G24 | 179 (Géluc.44/14) | 30 (Crémophor RH40) | 20 |
| G25 | 179 (Géluc.44/14) | 30 (Crémophor RH40) | 20 |
| G26 | 179 (Géluc.44/14) | 30 (Pluronic L44) | 20 |
| G27 | 179 (Géluc.44/14) | 30 (Pluronic L44) | 20 |
| G28 | 179 (Géluc.44/14) | 30 (tween 60) | 20 |
| G29 | 179 (Géluc.44/14) | 30 (Tween 60) | 20 |

Des gélules blanc opaque de taille 0 ont ensuite été fabriquées à l'aide des compositions des exemples ci-dessous et à l'aide de la composition du comparatif 2 permettant d'obtenir des gélules G1-G29 selon l'invention et une gélule non conforme à l'invention, i.e. gélule de référence. La composition du comparatif 1, non conforme à l'invention, a servi à fabriquer un comprimé non conforme à l'invention.

**Tableau 2**

| **Ingrédients** | **Composition Comparatif 1 (mg)** | **Composition Comparatif 2 (mg)** |
|---|---|---|
| Dronedarone sous forme de chlorhydrate | 426 | 213 |
| amidon prégélatinisé | 60.0 | 86,2 |
| lactose EFC | QS | 129,2 |
| talc | - | 48,0 |
| dioxide de silicone colloidal | 2.4 | 1,2 |
| stearate de magnésium | 6.0 | 2,4 |
| Hypromellose 6mPa.s | 12.0 | - |
| crospovidone | 30.0 | - |
| Poloxamer40 | 40 | - |
| Poids total (mg) | 640 | 480 |
| **Forme galénique** | **Comprimé 10%** | **Gélule de référence** |

Le matériel nécessaire pour la fabrication des compositions et des gélules, dont les modes opératoires sont décrits ci-dessous, est le suivant : Agitateur magnétique, Bécher, Balance de précision adaptée à la quantité pesée, Tamis 0,315 mm, Bain marie, Pipette Gilson 1000 µL à piston, Gélulier.

De plus, le gélucire 44/14 utilisé pour fabriquer les compositions est mis en étuve à 55°C la veille au soir de la fabrication. L'homogénéisation se fait manuellement par retournement du pot.

### Fabrication de la gélule G1 :

### Mode opératoire :

- Faire la pesée du gélucire® 44/14 préalablement fondu dans le bécher servant à la fabrication,
- La dronédarone sous forme de chlorhydrate est tamisée sur 0,315 mm d'ouverture de maille avant pesée,
- Fusion et mélange du gélucire® 44/14 et du Poloxamer 407 sous agitation lente à une vitesse d'agitateur de 200 rpm pendant une durée d'environ 10 min à une température du bain marie de 55°C-60°C,
- Addition du Propylène glycol à une vitesse agitateur de 200 rpm et à une température de bain marie de 55°C-60°C,
- Addition progressive et dispersion sous agitation vive du chlorhydrate de dronédarone préalablement tamisé à une vitesse pendant addition de 300-650 rpm. Après l'addition, mélanger pendant 30 min à une vitesse de mélange de 500 rpm et à une température de bain marie de 55°C-60°C,
- Remplissage avec une pipette automatique de gélules blanc opaque taille 0. Les gélules sont remplies par pesées unitaires. La vitesse d'agitation pendant la répartition est de 500 rpm et la température du bain marie est de 55°C-60°C,
- Après fermeture, les gélules sont disposées en position verticale sur le gélulier pour solidification à température ambiante.

### Fabrication de la gélule G2 :

### Mode opératoire :

- Faire la pesée du gélucire® 44/14 préalablement fondu dans le bécher servant à la fabrication,
- La dronédarone sous forme de chlorhydrate est tamisée sur 0,315 mm d'ouverture de maille avant pesée,
- Fusion et mélange du gélucire® 44/14 et du Poloxamer 407 sous agitation lente à une vitesse d'agitateur de 200 rpm pendant une durée d'environ 10 min à une température du bain marie de 55°C-60°C,
- Addition progressive et dispersion sous agitation vive du chlorhydrate de dronédarone préalablement tamisé à une vitesse pendant addition de 300-650 rpm. Après l'addition, mélanger pendant 10 min à une vitesse de mélange de 500 rpm à une température bain marie 55°C-60°C,
- Addition de la solution d'hydroxyde de sodium à 27% à une vitesse d'agitation de 500 rpm. Mélanger après addition pendant 30 min à une température de bain marie de 55°C-60°C,
- Remplissage avec une pipette automatique de gélules blanc opaque taille 0. Les gélules sont remplies par pesées unitaires. La vitesse d'agitation pendant la répartition est de 500 rpm et la température du bain marie est de 55°C-60°C,
- Après fermeture, les gélules sont disposées en position verticale sur le gélulier pour solidification à température ambiante.

### Fabrication de la gélule G3 :

### Mode opératoire :

- Faire la pesée du gélucire® 44/14 préalablement fondu dans le bécher servant à la fabrication,
- La dronédarone sous forme de chlorhydrate est tamisée sur 0,315 mm d'ouverture de maille avant pesée,
- Addition progressive et dispersion sous agitation vive du chlorhydrate de dronedarone préalablement tamisé à une vitesse pendant l'addition de 300-650 rpm. Après l'addition, mélanger pendant 30 min à une vitesse de mélange de 350 rpm et à une température de bain marie de 55°C-60°C,
- Remplissage avec une pipette automatique de gélules blanc opaque taille 0. Les gélules sont remplies par pesées unitaires. La vitesse d'agitation pendant la répartition est de 500 rpm et la température de bain marie de 55°C-60°C,
- Après fermeture, les gélules sont disposées en position verticale sur le gélulier pour solidification à température ambiante.

### Fabrication de la gélule G4 :

### Mode opératoire :

- Faire la pesée du gélucire® 44/14 préalablement fondu dans le bécher servant à la fabrication,
- La dronédarone est tamisée sur 0,315 mm d'ouverture de maille avant pesée,
- Fusion et mélange du gélucire® 44/14 et du Poloxamer 407 sous agitation lente à une vitesse d'agitateur de 200 rpm pendant une durée d'environ 10 min à une température du bain marie de 55°C-60°C,
- Addition progressive et solubilisation sous agitation vive de la dronédarone sous sa forme base préalablement tamisée à une vitesse pendant addition de 300-650 rpm. Après l'addition, mélanger pendant 30 min à une vitesse de mélange de 500 rpm et à une température de bain marie de 55°C-60°C,
- Addition progressive de l'eau sous agitation à une vitesse d'agitateur de 500 rpm pendant environ 10 min et à une température de bain marie de 55°C-60°C,
- Remplissage avec une pipette automatique de gélules blanc opaque taille 0. Les gélules sont remplies par pesées unitaires. La vitesse d'agitation pendant la répartition est de 500 rpm et la température de bain marie de 55°C-60°C,
- Après fermeture, les gélules sont disposées en position verticale sur le gélulier pour solidification à température ambiante.

### Fabrication de la gélule G5 :

### Mode opératoire :

- Faire la pesée du gélucire® 44/14 préalablement fondu dans le bécher servant à la fabrication,
- La dronédarone est tamisée sur 0,315 mm d'ouverture de maille avant pesée,
- Fusion et mélange du gélucire® 44/14 et du Poloxamer 407 sous agitation lente à une vitesse d'agitateur de 200 rpm pendant une durée d'environ 10 min à une température du bain marie de 55°C-60°C,
- Addition du Propylène glycol à une vitesse agitateur de 200 rpm et à une température de bain marie de 55°C-60°C,
- Addition progressive et solubilisation sous agitation vive de la dronédarone sous sa forme base préalablement tamisée à une vitesse pendant addition de 300-650 rpm. Après l'addition, mélanger pendant 30 min à une vitesse de mélange de 500 rpm et à une température de bain marie de 55°C-60°C,
- Remplissage avec une pipette automatique de gélules blanc opaque taille 0. Les gélules sont remplies par pesées unitaires. La vitesse d'agitation pendant la répartition est de 500 rpm et la température de bain marie de 55°C-60°C,
- Après fermeture, les gélules sont disposées en position verticale sur le gélulier pour solidification à température ambiante.

### Fabrication de la gélule G6 :

### Mode opératoire :

- Faire la pesée du gélucire® 44/14 préalablement fondu dans le bécher servant à la fabrication,
- La dronédarone est tamisée sur 0,315 mm d'ouverture de maille avant pesée,
- Fusion et mélange du gélucire® 44/14 sous agitation lente à une vitesse d'agitateur de 200 rpm pendant une durée d'environ 10 min à une température du bain marie de 55°C-60°C,
- Addition progressive et solubilisation sous agitation vive de la dronédarone sous sa forme base préalablement tamisée à une vitesse pendant addition de 300-650 rpm. Après l'addition, mélanger pendant 30 min à une vitesse de mélange de 500 rpm et à une température de bain marie de 55°C-60°C,
- Remplissage avec une pipette automatique de gélules blanc opaque taille 0. Les gélules sont remplies par pesées unitaires. La vitesse d'agitation pendant la répartition est de 500 rpm et la température de bain marie de 55°C-60°C,
Après fermeture, les gélules sont disposées en position verticale sur le gélulier pour solidification à température ambiante.

La fabrication de la gélule de référence se fait selon le même protocole que celui de la gélule G6 avec les proportions et les ingrédients tels qu'indiqués dans les tableaux 1 et 2 ci-dessus. Le comprimé constitué par la composition comparative 1 telle qu'indiquée dans le tableau 2 est fabriqué selon les techniques habituelles de fabrication de telles formes galéniques.

La fabrication des gélules G7 à G29 se fait selon le même protocole que celui de la gélule G6 avec les proportions et les ingrédients tels qu'indiqués dans les tableaux 3 et 4 ci-dessus.

### EVALUATION DE LA SOLUBILISATION DU PRINCIPE ACTIF DE LA COMPOSITION PHARMACEUTIQUE EN MILIEU AQUEUX AU COURS DU TRACTUS GASTRO-INTESTINAL

Afin de reproduire l'effet du pH sur le principe actif et en particulier sur sa dissolution au cours de son passage dans le tractus gastro-intestinal, un milieu gastro-intestinal a été mimé en reproduisant le pH de l'estomac puis le pH de l'intestin via un saut de pH. Une étude de la cinétique de dissolution a été menée en utilisant un test simple de dissolution in-vitro avec saut de pH.

### 000Principe :

Le principe consiste à déterminer la solubilisation du principe actif formulé en étudiant sa cinétique de dissolution à 37°C, d'abord en milieu gastrique simulé pH 4, ensuite en milieu intestinal simulé à pH 6,5 et ce dans un intervalle de temps compatible avec le tractus gastro-intestinal.

### • 000Matériel et méthode

*000Matériel :* balance de précision (Mettler AE200 ou AT261), pH-mètre (Knick ou Schott Geräte ou Inolab), dissolutest thermostaté 6 ou 7 bols (Sotax AT6 ou AT7), filtre 5 µm (PALL versapore 25 mm) avec seringue (Térumo), spectrophotomètre UV (Gilford Response II ou Perkin Elmer) ou HPLC (Merck ou Agilent)..

*Milieux de dissolution :* Les milieux physiologiques simulés représentatifs du tractus gastro-intestinal sont obtenus à partir de fluides gastriques et intestinaux simulés préconisés par l'USP, mais utilisés sans pepsine ni pancréatine.

### • fluide gastrique simulé - USP (sans pepsine) :

- 2 g de chlorure de sodium pour 900 ml d'eau distillée,
- pH ajusté à 1,2 avec de l'acide chlorhydrique concentré (37%),
- QSP 1000 ml avec de l'eau distillée.

### • fluide intestinal simulé - USP (sans pancréatine) :

- 6,8 g de potassium dihydrogénophosphate pour 900 ml d'eau distillée,
- pH ajusté à 7,5 avec hydroxyde de sodium concentré (10 M),
- QSP 1000 ml avec de l'eau distillée.

Le milieu gastrique simulé de pH 4 est ainsi obtenu par mélange dans des proportions différentes des deux fluides simulés et sous contrôle d'une électrode de pH.

Le milieu de dissolution type intestinal est ajusté à pH 6,5 avec quelques gouttes de soude concentrée (10M) pour simuler le passage du principe actif dans le début de l'intestin, sans entraîner de dilution.

### Méthode :

- **Etalonnage :** Des solutions étalons de principe actif sont préparées dans un milieu solvant, préférentiellement dans la phase mobile ou l'éthanol, méthanol, puis dosées à la longueur d'onde caractéristique du principe actif. Une droite d'étalonnage représentative des concentrations en fonction des densités optiques (dosage spectrophotométrie UV) ou des aires sous pic (dosage HPLC) est alors déterminée. L'équation de la droite obtenue permet de déterminer la concentration de principe actif solubilisé à partir de la mesure de la densité optique ou de l'aire sous pic.
- **000Cinétiques de solubilisation :** La concentration mise en oeuvre correspond à la dose dans 250 ml (soit 4 gélules dans un bol de 500 ml). Les cinétiques de solubilisation sont d'abord étudiées en milieu gastrique simulé pH 4 en dissolutest dans des bols thermostatés à 37°C, sous agitation par pâles à 75 rpm, pendant 1 heure avec des prélèvements aux temps 5, 15, 30, 45 et 60 minutes, puis en milieu intestinal avec une remontée du pH à 6,5 par ajout d'un faible volume de soude concentrée (exemple : 0,400 ml pour 500 ml de milieu pH 4), sous contrôle d'une électrode de pH. La cinétique est poursuivie jusqu'à 3 heures avec des prélèvements aux temps 75, 90, 120 et 180 minutes et chaque prélèvement est filtré sur 5 µm puis dosé.
- **Dosages :** Deux méthodes de dosage peuvent être utilisées selon la sensibilité requise et la forme galénique étudiée : spectrophotométrie UV (pour principe actif seul) ou HPLC (pour principe actif seul ou formulé).

La concentration à un temps t est déterminée à l'aide de l'étalonnage établi au préalable. Lors d'un dosage UV par spectrophotométrie, le spectre d'absorption complet est contrôlé au moins en fin de cinétique. De même, le pH du milieu est contrôlé en fin de cinétique.

### 000Resultats :

La cinétique de solubilisation du principe actif (exprimée en pourcentage de produit libéré) en fonction du temps est tracée d'abord à pH 4 (simulation du milieu gastrique), puis à pH 6,5 (simulation du milieu intestinal) en continuité sur une même courbe représentée en **Figure 1.** La cinétique de solubilisation a été mesurée dans les mêmes conditions pour les gélules G1-G7 et pour la gélule de référence dont les compositions ont été définies ci-dessus

Le comprimé présente une amélioration significative mais sensible du % de principe actif libéré par rapport à la gélule de référence mais seulement en conditions de pH intestinal simulé.

La gélule G3 contenant du chlorhydrate de dronedarone dans une matrice d'excipient lipidique en absence de tensio-actif présente une amélioration significative du % de principe actif libéré par rapport à la gélule de référence ou au comprimé avec en plus une meilleure libération dans les conditions de pH gastrique simulé.

La gélule G1 contenant du chlorhydrate de dronedarone dans une matrice d'excipient lipidique en présence de tensio-actif présente une amélioration du % de principe actif libéré très significative par rapport à la gélule G3 et par rapport à la gélule de référence et le comprimé.

La gélule G2 contenant de la dronedarone base formée in situ dans la matrice d'excipient lipidique à partir du Chlorhydrate de dronedarone présente une amélioration très significative du % de principe actif libéré par rapport à la gélule de référence, au comprimé et à la gélule G3.

Les gélules G4 et G5 contenant de la dronédarone sous forme de base libre dans une matrice d'excipient lipidique présentent aussi une amélioration très significative du % de principe actif libéré par rapport à la gélule de référence, au comprimé et à la gélule G3 avec en outre un comportement au cours de la dissolution équivalent à celui de la gélule G2.

On constate, au vu des courbes représentées sur la **Figure 1,** l'effet bénéfique de la présence de l'excipient lipidique dans les formulations contenant la dronédarone sous forme de base ou de chlorhydrate par rapport à la gélule de référence et au comprimé qui en sont exempts.

D'autre part, on constate une très nette amélioration du % de principe actif libéré pour les gélules comprenant en outre du tensioactif dans leur composition par rapport aux gélules qui en sont dépourvues (courbe de G1 vs courbe de G3 et courbe de G5 vs courbe de G6).

De plus, dans le cas des formulations contenant de la dronédarone sous sa forme base dans la matrice, le profil de % de principe actif libéré est rapide dès les premiers instants dans les conditions de pH gastrique simulé à la différence de formulation contenant le chlorhydrate de dronédarone dont la libération s'observe dans le meilleur des cas à pH intestinal.

### Impact du taux de Gélucire 44/14 sur la forme base de dronedarone

On constate au vu des courbes présentées sur la figure 2, l'effet positif de l'augmentation du taux de gélucire.

### Impact du taux de Gélucire 44/14 sur le sel de chlorhydrate de dronedarone

On constate au vu des courbes présentées sur la figure 3, l'effet positif de l'augmentation du taux de gélucire.

### Impact du taux de tensioactif sur la forme base de dronedarone

On constate au vu des courbes présentées sur la figure 4 que le taux de tensioactif dans une composition selon l'invention n'a pas d'impact sur la libération du principe actif.

### Impact du taux de tensioactif sur le sel de chlorhydrate de dronedarone

On constate au vu des courbes présentées sur la figure 5 que le taux de tensioactif dans une composition selon l'invention a un effet bénéfique sur la libération du principe actif avec un taux optimal entre 10 et 30%.

### Influence de la nature du tensioactif sur la forme base de dronedarone et sur le chlorhydrate de dronedarone

| Surfactant | HLB | % de HCl de dronedaron e à 180 min | % dronedarone forme base à 180 min |
|---|---|---|---|
| Pluronic L44 | 12 | 49,1 | 76,38 |
| Chrem RH40 | 14 | 59,01 | 85,3 |
| Tween 60 | 15 | 58,32 | 81,12 |
| Polox 407 | 22 | 90,49 | 72,89 |
| Polox 188 | 29 | 69,82 | 71,32 |

On constate au vu des courbes présentées sur la figure 6 que les cinétiques de libération de la forme base sont équivalentes quelque soit le tensioactif non ionique utilisé.

On constate au vu des courbes présentées sur la figure 7 que les cinétiques de libération du chlorhydrate de dronedarone présentent de meilleurs profils pour un HLB entre 15 et 25 et plus particulièrement autour de 22.

### EVALUATION DE LA BIODISPONIBILITE

La biodisponibilité est relative à la quantification de l'absorption du médicament. Elle est liée à la fraction de la dose de médicament administré qui atteint la circulation générale et à la vitesse à laquelle elle l'atteint. La biodisponibilité pour une prise orale dépend de l'absorption digestive et du métabolisme pré-systémique dans l'intestin et le foie entre autres.

*Protocole :* 12 sujets jeunes et en bonne santé reçoivent soit à jeun soit pendant un repas riche en graisses une dose unique de 400 mg BID de dronedarone en absorbant les gélules G1, G2 ou G3 de compositions définie ci-dessus. Des échantillons de sang sont collectés régulièrement pendant 48h et le plasma recueilli est testé avec des méthodes LC-UV afin de déterminer en fonction du temps la concentration de dronédarone dans le plasma.

Le Cmax, Tmax et l'AUC sont mesurés sur les courbes ainsi obtenues. Les résultats obtenus sont regroupés dans les tableaux 4 et 5 suivants.

**Cmax** correspond au pic de concentration plasmatique de dronédarone.

**tmax** correspond au temps permettant d'obtenir le Cmax.

**AUC** correspond l'aire sous la courbe ou Intégrale de Concentration plasmatique en fonction du temps t.

**Tableau 5**

| **Paramètres** | **Gélule G1 / gélule référence** | **Gélule G2 / gélule référence** | **Gélule G3 / gélule référence** |
|---|---|---|---|
| **À jeun** | | | |
| Cₘₐₓ * | 4.57 | 8.22 | 2.57 |
| AUC ** | 8.41 | 16.5 | 3.92 |

| **Avec repas** | | | |
|---|---|---|---|
| Cₘₐₓ *** | 1.51 | 1.57 | 1.20 |
| AUC**** | 1.45 | 1.47 | 1.22 |

| | | | |
|---|---|---|---|
| * **Cmax (à jeun)** correspond au ratio entre le Cmax mesuré pour une Gélule selon l'invention absorbée par un patient à jeun et le Cmax mesuré pour une Gélule de référence, absorbée par ce même patient à jeun. ** **AUC (à jeun)** correspond au ratio entre l'AUC mesurée pour une Gélule selon l'invention absorbée par un patient à jeun et l'AUC mesurée pour une Gélule de référence, absorbée par ce même patient à jeun. *** **Cmax (avec repas)** correspond au ratio entre le Cmax mesuré pour une Gélule selon l'invention absorbée par un patient pendant un repas et le Cmax mesuré pour une Gélule de référence, absorbée par ce même patient pendant un repas. **** **AUC (avec repas)** correspond au ratio entre l'AUC mesurée pour une Gélule selon l'invention absorbée par un patient pendant un repas et l'AUC mesurée pour une Gélule de référence, absorbée par ce même patient pendant un repas. | | | |

**Tableau 6**

| **Paramètres** | **Gélule de référence (Avec repas / à jeûn)** | **Gélule G1 (Avec repas / à jeûn)** | **Gélule G2 (Avec repas / à jeûn)** | **Gélule G3 (Avec repas / à jeûn)** |
|---|---|---|---|---|
| Cₘₐₓ¤ | 6.60 | 2.18 | 1.26 | 3.08 |
| Effet repas¤¤ | 16.5 | 2.83 | 1.46 | 5.12 |

| | | | | |
|---|---|---|---|---|
| ¤ **Cmax** correspond au ratio entre le Cmax mesuré pour une Gélule donnée absorbée par un patient pendant le repas et le Cmax mesuré pour une même Gélule donnée absorbée par un patient à jeun. **¤¤ Effet repas** correspond au ratio entre l'AUC mesurée pour une Gélule donnée absorbée par un patient pendant le repas et l'AUC mesurée pour une même Gélule donnée absorbée par un patient à jeun. | | | | |

### Résultats :

Les résultats indiquent qu'en conditions à jeun, la biodisponibilité des gélules G1, G2, G3 selon l'invention, augmente de façon significative comparé à la gélule de référence, la gélule G2 étant la plus efficace.

On constate de plus que l'effet repas baisse de façon significative pour les gélules selon l'invention par rapport à la gélule de référence, la gélule G2 étant celle qui présente l'effet repas le plus bas de l'ordre de 1,46.

### EVALUATION DE LA BIODISPONIBILITE

### Protocole

### Traitement et administration

La dose utilisée est de 60 mg/animal quelque soit la période/condition correspondant à 6 mg/kg, (assumant un poids de 10 kg pour un chien) et à la dose de 400 mg donnée chez l'homme (c'est à dire environ 6 mg/kg pour un poids humain de 70 kg).

Les conditions d'administration sont les suivantes :
- Période à jeun: les animaux ne sont pas nourris la nuit précédent le dosage. De l'eau ainsi que la nourriture de routine (SSNIFFhdH) sont donnés respectivement une heure et 4 heures après administration.
- Période nourri: les animaux reçoivent 50g d'un régime riche en graisse (SSNIFF EF Dog FDA Model high fat), 10 minutes avant dosage (ce régime à une valeur énérgetique de 100 kcal et est composé à 15% de protéines, 25% de carbohydrates et de 50-60% de graisse). De l'eau et la nourriture de routine pour chien (SSNIFFhdH) sont ensuite donnés respectivement une heure et 4 heures après administration.

Un prétraitement avec de la pentagastrine est conduit 0.5 heure avant dosage. La pentagastrine (6 µg/kg, 0.25 mL/kg) est administrée en intra musculaire et permet de maintenir le pH gastrique de l'animal entre 2-3, mimant ainsi les conditions humaines.

Le dosage avec la capsule est suivi de 30 mL d'eau par gavage ce qui correspond approximativement à un volume de 240 mL donné à un sujet humain lors d'un essai clinique.

Les traitements sont:
Traitement 1: 60 mg d'hydrochlorate de dronedarone en capsule en conditions à jeun, voie orale (capsule de référence) (ref 2)
Traitement 2 : 60 mg de chlorhydrate de dronedarone en capsule avec du gélucire et du poloxamère 407, en conditions à jeun, voie orale (=G1).
Traitement 3 : 60 mg de dronedarone forme base reconstituée in situ à partir de chlorhydrate de dronedarone en capsule avec du gélucire et du poloxamère 407, en conditions à jeun, voie orale (=G2).
Traitement 4 : 60 mg de dronedarone forme base en capsule avec du gélucire et du poloxamère 407, en conditions à jeun, voie orale (= G5).
Traitement 5 : 60 mg de dronedarone forme base en capsule avec du gélucire et du poloxamère 407, en conditions nourri, voie orale (= G5).
Traitement 6 : 60 mg de dronedarone forme base en capsule avec gélucire et sans poloxamère 407, en conditions à jeun, voie orale (=G8).
Traitement 7 : 60 mg de dronedarone forme base en capsule avec gélucire et sans poloxamère 407, en conditions nourri, voie orale (=G8).

### Prélèvements et analyses

Les échantillons de sang sont prélevés, dans des tubes en plastique contenant de l'héparine de lithium comme anticoagulant, aux temps de prélèvement suivants : avant traitement et 0.5, 1, 2, 3, 4, 6, 8 et 24 heures après administration de chaque traitement.

La concentration plasmatique de dronedarone est déterminée en utilisant une méthode de dosage exploratoire par chromatographie liquide couplée à un spectromètre de masse (LC-MS/MS). La limite basse de détection avec cette méthode pour les composés testés est de 0,5 ng/mL.

### Expression des résultats

Les paramètres pharmacocinétiques sont calculés à partir des concentrations individuelles par une analyse non compartimentale en utilisant le logiciel WinNonLin 5.2.1 (Pharsight, USA) et en utilisant les temps de prélèvement théoriques (à condition que les temps réels de prélèvement ne diffèrent pas de plus de 15 % des temps théoriques).

Les paramètres pharmacocinétiques suivants ont été mesurés pour chaque traitement:
- Cₘₐₓ (ng/mL): correspond à la concentration plasmatique maximale observée,
- tₘₐₓ (h): correspond au temps observé permettant d'obtenir la concentration maximale,
- AUCₗₐₛₜ : correspond à l'aire sous la courbe ou intégrale de la concentration plasmatique en fonction du temps t calculé par la méthode des trapèzes de t₀ jusqu'au temps correspondant à la dernière concentration quantifiable.
- AUC : correspond à l'aire sous la courbe ou intégrale de concentration plasmatique en fonction du temps extrapolée à l'infini.
- T1/2z ; demi-vie d'élimination terminale

Les paramètres suivants ont aussi été évalués :
- biodisponibilité relative sur Cmax et AUC
- rapport de d'effet repas sur Cmax et AUC.

### Résultats

**Tableau 7 -Paramètres pharmacocinétiques de dronedarone (Mean±SD (CV%)) dans le groupe 1 (n=4 par formulation)**

| **Traitement** | **Cₘₐₓ (ng/mL)** | **tₘₐₓ (h)*** | **AUCₗₐₛₜ (ng.h/mL)** | **AUC (ng.h/mL)** | **t_{1/2z} (h)** |
|---|---|---|---|---|---|
| capsule de référence avec HCl de | 5.73±4.57 (80%) | 2.50 (0.50-3.00) | 18.9±14.2 (75%) | 21.2±14.4 (68%) | 1.88±0.624 (33%) |
| dronedarone et poloxamer à jeun | | | | | |
| Capsule avec Gelucire, HCl de dronedarone et avec poloxamer à jeun | 13.5±4.87 (36%) | 1.50 (0.50-2.00) | 45.0±17.8 (40%) | 51.3±21.2 (41%) | 2.53±0.377 (15%) |
| capsule avec Gelucire, dronedarone forme base reconstitutée et poloxamer à jeun | 19.5±13.0 (67%) | 1.00 (0.50-1.00) | 53.3±33.4 (63%) | 60.5±35.7 (59%) | 2.70±0.762 (28%) |

| | | | | | |
|---|---|---|---|---|---|
| *median (min-max) | | | | | |

**Tableau 8 - Paramètres pharmacocinétiques de dronedarone (Mean±SD (CV%)) dans le groupe 2 (n=4 par formulation)**

| **Traitement** | **Cₘₐₓ (ng/mL)** | **tₘₐₓ(h)*** | **AUClast (ng.h/mL)** | **AUC (ng.h/mL)** | **t_{1/2z} (h)** |
|---|---|---|---|---|---|
| capsule de référence avec HCl de dronedarone et poloxamer à jeun | 7.36±4.83 (66%) | 1.00 (0.50-2.00) | 21.5±13.9 (65%) | 28.1±18.0 (64%)* | 3.07±0.153 (5%)* |
| capsule avec Gelucire, dronedarone forme base et poloxamer à jeun | 24.6±14.8 (60%) | 1.00 (1.00-2.00) | 62.3±34.0 (55%) | 69.0±37.7 (55%) | 2.40±0.535 (22%) |
| capsule avec Gelucire, dronedarone forme base et poloxamer nourri | 16.9±7.41 (44%) | 1.00 (1.00-2.00) | 44.0±19.9 (45%) | 48.0±21.5 (45%) | 2.10±0.183 (9%) |

| | | | | | |
|---|---|---|---|---|---|
| n=3; * median (min-max) | | | | | |

**Tableau 9**

| **Traitement** | **Cₘₐₓ (ng/mL)** | **tₘₐₓ (h)*** | **AUClast (ng.h/mL)** | **AUC (ng.h/mL)** | **t_{1/2z} (h)** |
|---|---|---|---|---|---|
| capsule de référence avec HCl de dronedarone et poloxamer à jeun | 4.52±3.04 (67%) | 1.50 (0.50-6.00) | 15.3±8.62 (56%) | 15.6±7.73 (50%) | 2.90±0.794 (27%) |
| capsule avec Gelucire, dronedarone forme base sans poloxamer à jeun | 15.6±4.99 (32%) | 1.00 (1.00-2.00) | 57.5±11.3 (20%) | 66.8±10.1 (15%) | 2.68±0.377 (14%) |
| capsule avec Gelucire, | 30.7±12.7 (41%) | 1.00 (0.50-2.00) | 82.8±29.1 (35%) | 91.8±31.5 (34%) | 2.53±0.171 (7%) |
| dronedarone forme base sans poloxamer nourri | | | | | |

| | | | | | |
|---|---|---|---|---|---|
| * médian (min-max) | | | | | |

Tous les chiens recevant la formulation de référence présentent une exposition similaire en conditions à jeun quelque soit le groupe.

**Tableau 10- Biodisponibilité relative de dronedarone (%) avec 90% CI en conditions à jeun (utilisant la capsule comme référence)**

| **Traitement** | **Cₘₐₓ** | **AUCₗₐₛₜ** | **AUC** |
|---|---|---|---|
| capsule avec Gelucire, HCl de dronedarone avec poloxamer à jeun | 274 (120-625) | 283 (112-716) | 271 (119-613) |
| capsule avec Gelucire base reconstitutée avec poloxamer à jeun | 357 (157-812) | 311 (123-785) | 301 (133-682) |
| Capsule avecGelucire et dronedarone forme base avec poloxamer à jeun | 339 (159-724) | 325 (177-595) | 248 (134-459)* |
| Capsule avecGelucire et dronedarone forme base sans poloxamer à jeun | 401 (230-700) | 432 (240-778) | 500 (273-915) |

| | | | |
|---|---|---|---|
| n=3 for 2B1 | | | |

Toutes les formulations testées présentent une biodisponibilité plus importante que la capsule de référence avec une biodisponibilité relative allant de 271% à 500% en conditions à jeun.

Les formulations avec gélucire avec le chlohydrate de dronedarone et reconstituant la base in situ (Frel=301%) ont montré une biodisponibilité plus importante que la capsule de référence comme dans l'essai clinique décrit ci-dessus.

Les formulations avec gelucire utilisant la base native exhibe une biodisponibilité relative similaire à la formulation gélucire utilisant le chlorhydrate de dronedarone et reconstituant la base in situ quand comparé à la référence en conditions à jeun comme indiqué par le recouvrement de l'intervalle de confiance.

Les formulations avec gelucire avec ou sans poloxamère montre une biodisponibilité relative similaire avec une biodisponibilité supérieure de 3 à 5 comparé à la capsule de référence.

**Tableau 11- Rapport d'effet repas pour la capsule avec gelucire, dronedarone forme base avec poloxamère**

| **Traitement** | **Cₘₐₓ** | **AUCₗₐₛₜ** | **AUC** |
|---|---|---|---|
| nourri/à jeun | 0.73 (0.35-1.53) | 0.71 (0.41-1.23) | 0.70 (0.41-1.21) |

Il y a une tendance à une légère baisse de Cmax de 1,4 fois quand la capsule gelucire avec poloxamère est administrée avec de la nourriture riche en graisse. Cette baisse n'est pas significative car le 90% CI inclus l'unité.

**Tableau 12- Rapport d'effet repas pour la capsule avec gelucire, dronedarone forme base sans poloxamer**

| **Traitement** | **Cₘₐₓ** | **AUCₗₐₛₜ** | **AUC** |
|---|---|---|---|
| nourri/à jeun | 1.93 (1.16-3.21) | 1.39 (0.82-2.35) | 1.32 (0.81-2.16) |

Il y a une tendance à un effet repas positif quand la capsule gelucire sans poloxamère est administrée avec une nourriture riche en graisse. En effet le Cmax est augmentée de 1,9 fois, AUClast par 1,4 fois et un AUC par 1,3 fois. Cependant, cette augmentation n'est pas significative concernant l'AUC comme le 90% CI inclus l'unité.

## Revendications

1. Composition pharmaceutique comprenant au moins un principe actif choisi parmi le (i) 2-n-butyl-3-[4-(3-di-n-butylaminopropoxy)benzoyl]-5-methylsulfonamido-benzofurane sous forme de base, (ii) le 2-n-butyl-3-[4-(3-di-n-butylaminopropoxy)benzoyl]-5-methylsulfonamido-benzofurane sous forme d'un sel pharmaceutiquement acceptable, **caractérisée en ce qu'**elle comprend en outre au moins un excipient lipidique amphiphile de valeur HLB comprise entre 5 et 18 et **en ce que** ledit excipient lipidique est choisi parmi les polyoxylglycerides substitués semi-solides .

2. Composition selon la revendication précédente, **caractérisée en ce que** le 2-n-butyl-3-[4-(3-di-n-butylaminopropoxy)benzoyl]-5-methylsulfonamido-benzofurane sous forme d'un sel pharmaceutiquement acceptable est choisi parmi le chlorhydrate de 2-n-butyl-3-[4-(3-di-n-butylaminopropoxy)benzoyl]-5-methylsulfonamido-benzofurane, le fumarate de 2-n-butyl-3-[4-(3-di-n-butylaminopropoxy)benzoyl]-5-methylsulfonamido-benzofurane et l'oxalate de 2-n-butyl-3-[4-(3-di-n-butylaminopropoxy)benzoyl]-5-methylsulfonamido-benzofurane.

3. Composition selon l'une quelconque des revendications précédentes, **caractérisé en ce que** le principe actif est choisi parmi le 2-n-butyl-3-[4-(3-di-n-butylaminopropoxy)benzoyl]-5-methylsulfonamido-benzofurane sous forme de base et le chlorhydrate de 2-n-butyl-3-[4-(3-di-n-butylaminopropoxy)benzoyl]-5-methylsulfonamido-benzofurane.

4. Composition selon l'une quelconque des revendications précédentes, **caractérisé en ce que** ladite composition comprend en outre au moins un tensio-actif et/ou au moins un co-solvant.

5. Composition selon l'une quelconque des revendications précédentes, **caractérisé en ce que** ledit excipient lipidique amphiphile de valeur HLB comprise entre 5 et 18 a un point de fusion inférieur à 50°C.

6. Composition selon l'une quelconque des revendications précédentes,**caractérisé en ce qu'**elle comprend le chlorhydrate de 2-n-butyl-3-[4-(3-di-n-butylaminopropoxy)benzoyl]-5-méthylsulfonamidobenzofurane, comme principe actif.

7. Composition selon l'une quelconque des revendications précédentes, **caractérisé en ce que** ledit excipient lipidique amphiphile de valeur HLB comprise entre 5 et 18 est choisi parmi le lauroyl macroglyceride commercialisé sous la marque Gélucire® 44/14 et le Gélucire® 50/13.

8. Composition selon l'une quelconque des revendications précédentes, **caractérisé en ce que** ledit excipient lipidique amphiphile de valeur HLB comprise entre 5 et 18 est le lauroyl macroglyceride commercialisé sous la marque Gélucire® 44/14.

9. Composition selon l'une des revendications précédentes, **caractérisé en ce qu'**elle comprend :
• 1-60% en poids d'au moins un principe actif ;
• 40-99% en poids d'au moins un excipient lipidique ;
• 0-30% d'au moins un composé choisi parmi les tensio-actifs, les co-solvants, les diluants, les désintégrants, les lubrifiants, les bases organiques ou inorganiques et les plastifiants,
les % étant exprimés en poids par rapport au poids total de ladite composition.

10. Composition selon l'une des revendications précédentes, **caractérisé en ce qu'**elle comprend :
• 1-60% en poids d'au moins un principe actif;
• 40-99% en poids d'au moins un excipient lipidique,
• 0-30% en poids d'au moins un tensio-actif,et
• 0-29% en poids d'au moins un co-solvant;
les % étant exprimés en poids par rapport au poids total de ladite composition.

11. Composition selon l'une des revendications 9 ou 10, **caractérisé en ce qu'**elle comprend :
• 1-50% en poids d'au moins un principe actif, avantageusement entre 10 et 45%, mieux encore entre 20% et 40% ;
et/ou
• 45-80% en poids d'au moins un excipient lipidique, avantageusement entre 50% et 60% ;
et/ou
• 1-20% en poids d'au moins un tensio-actif, avantageusement entre 5% et 15% ; et/ou
• 1-20% en poids d'au moins un co-solvant, avantageusement entre 2 et 15%.

12. Composition selon l'une des revendications 9 à 11, **caractérisé en ce que** le tensioactif est hydrophile non ionique.

13. Composition selon l'une des revendications 9 à 12, **caractérisé en ce que** le tensioactif est choisi parmi:
• des copolymères oxyde d'éthylène/oxyde de propylène ;
• des huiles de ricin polyéthoxylées ;
• des polysorbates éthoxylés, et
• des hydroxystéarates de polyéthylène.

14. Composition selon l'une quelconque des revendications 9 à 13, **caractérisé en ce que** le tensioactif est le poloxamer 407.

15. Composition selon l'une quelconque des revendications 9 à 14, **caractérisé en ce que** le co-solvant est choisi parmi les solvants organiques alcooliques ou les dérivés de glycol.

16. Forme galénique comprenant une composition selon l'une quelconque des revendications 1 à 15.

17. Forme galénique selon la revendication 16, **caractérisée en ce qu'**il s'agit d'une capsule choisie parmi les gélules, les capsules à enveloppe molle, les capsules gastrorésistantes et les capsules à libération modifiée.

18. Forme galénique selon l'une quelconque des revendications 16 à 17, **caractérisée en ce qu'**il s'agit d'une gélule.

19. Forme galénique selon l'une quelconque des revendications 16 à 18, **caractérisée** en que qu'elle contient entre 50 et 500 mg de principe actif, avantageusement entre 200 à 400 mg de principe actif.

## Patentansprüche

1. Pharmazeutische Zusammensetzung, die mindestens einen Wirkstoff, ausgewählt aus (i) 2-n-Butyl-3-[4-(3-di-n-butylaminopropoxy)benzoyl]-5-methylsulfonamidobenzofuran in Basenform, (ii) 2-n-Butyl-3-[4-(3-di-n-butylaminopropoxy)benzoyl]-5-methylsulfonamidobenzofuran in Form eines pharmazeutisch unbedenklichen Salzes, umfasst, **dadurch gekennzeichnet, dass** sie weiterhin mindestens einen amphiphilen Lipidgrundstoff mit einem HLB-Wert zwischen 5 und 18 umfasst und dadurch, dass der Lipidgrundstoff aus halbfesten substituierten Polyoxylglyceriden ausgewählt ist.

2. Zusammensetzung nach dem vorhergehenden Anspruch, **dadurch gekennzeichnet, dass** das 2-n-Butyl-3-[4-(3-di-n-butylaminopropoxy)benzoyl]-5-methylsulfonamidobenzofuran in Form eines pharmazeutisch unbedenklichen Salzes aus 2-n-Butyl-3-[4-(3-di-n-butylaminopropoxy)benzoyl]-5-methylsulfonamidobenzofuran-Hydrochlorid, 2-n-Butyl-3-[4-(3-di-n-butylaminopropoxy)benzoyl]-5-methylsulfonamidobenzofuran-Fumarat und 2-n-Butyl-3-[4-(3-di-n-butylaminopropoxy)benzoyl]-5-methylsulfonamidobenzofuran-Oxalat ausgewählt ist.

3. Zusammensetzung nach einem der vorhergehenden Ansprüche, **dadurch gekennzeichnet, dass** der Wirkstoff aus 2-n-Butyl-3-[4-(3-di-n-butylaminopropoxy)benzoyl]-5-methylsulfonamidobenzofuran in Basenform und aus 2-n-Butyl-3-[4-(3-di-n-butylaminopropoxy)benzoyl]-5-methylsulfonamidobenzofuran-Hydrochlorid ausgewählt ist.

4. Zusammensetzung nach einem der vorhergehenden Ansprüche, **dadurch gekennzeichnet, dass** die Zusammensetzung weiterhin mindestens ein Tensid und/oder mindestens ein Hilfslösungsmittel umfasst.

5. Zusammensetzung nach einem der vorhergehenden Ansprüche, **dadurch gekennzeichnet, dass** der amphiphile Lipidgrundstoff mit einem HLB-Wert zwischen 5 und 18 einen Schmelzpunkt unter 50°C aufweist.

6. Zusammensetzung nach einem der vorhergehenden Ansprüche, **dadurch gekennzeichnet, dass** sie 2-n-Butyl-3-[4-(3-di-n-butylaminopropoxy)benzoyl]-5-methylsulfonamidobenzofuran-Hydrochlorid als Wirkstoff umfasst.

7. Zusammensetzung nach einem der vorhergehenden Ansprüche, **dadurch gekennzeichnet, dass** der amphiphile Lipidgrundstoff mit einem HLB-Wert zwischen 5 und 18 aus Lauroylmacroglycerid, das unter dem Handelsnamen Gélucire® 44/14 vertrieben wird, und Gélucire® 50/13 ausgewählt ist.

8. Zusammensetzung nach einem der vorhergehenden Ansprüche, **dadurch gekennzeichnet, dass** der amphiphile Lipidgrundstoff mit einem HLB-Wert zwischen 5 und 18 das unter dem Handelsnamen Gélucire®44/14 vertriebene Lauroylmacroglycerid ist.

9. Zusammensetzung nach einem der vorhergehenden Ansprüche, **dadurch gekennzeichnet, dass** sie Folgendes umfasst:
• 1-60 Gew.-% an mindestens einem Wirkstoff;
• 40-99 Gew.-% an mindestens einem Lipidgrundstoff;
• 0-30% an mindestens einer Verbindung, ausgewählt aus Tensiden, Hilfslösungsmitteln, Verdünnungsmitteln, Zerfallhilfsmitteln, Schmiermitteln, organischen oder anorganischen Basen und Weichmachern,
wobei die % als Gewicht in Bezug auf das Gesamtgewicht der Zusammensetzung ausgedrückt sind.

10. Zusammensetzung nach einem der vorhergehenden Ansprüche, **dadurch gekennzeichnet, dass** sie Folgendes umfasst:
• 1-60 Gew.-% an mindestens einem Wirkstoff;
• 40-99 Gew.-% an mindestens einem Lipidgrundstoff;
• 0-30 Gew.-% an mindestens einem Tensid und
• 0-29 Gew.-% an mindestens einem Hilfslösungsmittel,
wobei die % als Gewicht in Bezug auf das Gesamtgewicht der Zusammensetzung ausgedrückt sind.

11. Zusammensetzung nach einem der Ansprüche 9 oder 10, **dadurch gekennzeichnet, dass** sie Folgendes umfasst:
• 1-50 Gew.-% an mindestens einem Wirkstoff, vorteilhafterweise zwischen 10 und 45%, noch besser zwischen 20% und 40%;
und/oder
• 45-80 Gew.-% an mindestens einem Lipidgrundstoff, vorteilhafterweise zwischen 50% und 60%;
und/oder
• 1-20 Gew.-% an mindestens einem Tensid, vorteilhafterweise zwischen 5% und 15%;
und/oder
• 1-20 Gew.-% an mindestens einem Hilfslösungsmittel, vorteilhafterweise zwischen 2 und 15%.

12. Zusammensetzung nach einem der Ansprüche 9 bis 11, **dadurch gekennzeichnet, dass** das Tensid hydrophil, nichtionisch ist.

13. Zusammensetzung nach einem der Ansprüche 9 bis 12, **dadurch gekennzeichnet, dass** das Tensid aus den Folgenden ausgewählt ist:
• Ethylenoxid/Propylenoxid-Copolymeren;
• polyethoxylierten Rizinusölen;
• ethoxylierten Polysorbaten und
• Polyethylenhydroxystearaten.

14. Zusammensetzung nach einem der Ansprüche 9 bis 13, **dadurch gekennzeichnet, dass** das Tensid Poloxamer 407 ist.

15. Zusammensetzung nach einem der Ansprüche 9 bis 14, **dadurch gekennzeichnet, dass** das Hilfslösungsmittel aus alkoholischen organischen Lösungsmitteln oder Glykolderivaten ausgewählt ist.

16. Darreichungsform, die eine Zusammensetzung nach einem der Ansprüche 1 bis 15 umfasst.

17. Darreichungsform nach Anspruch 16, **dadurch gekennzeichnet, dass** es sich um eine Kapsel, ausgewählt aus Hartkapseln, Kapseln mit weicher Hülle, magensaftresistenten Kapseln und Kapseln mit modifizierter Freisetzung, handelt.

18. Darreichungsform nach einem der Ansprüche 16 bis 17, **dadurch gekennzeichnet, dass** es sich um eine Hartkapsel handelt.

19. Darreichungsform nach einem der Ansprüche 16 bis 18, **dadurch gekennzeichnet, dass** sie zwischen 50 und 500 mg Wirkstoff, vorteilhafterweise zwischen 200 und 400 mg Wirkstoff, enthält.

## Claims

1. Pharmaceutical composition comprising at least one active principle selected from (i) 2-n-butyl-3-[4-(3-di-n-butylaminopropoxy)benzoyl]-5-methylsulfonamidobenzofuran in the form of base, (ii) 2-n-butyl-3-[4-(3-di-n-butylaminopropoxy)benzoyl]-5-methylsulfonamidobenzofuran in the form of a pharmaceutically acceptable salt, **characterized in that** it further comprises at least one amphiphilic lipid excipient with HLB value between 5 and 18 and **in that** said lipid excipient is selected from the semi-solid subsituted polyoxylglycerides.

2. Composition according to the preceding claim, **characterized in that** the 2-n-butyl-3-[4-(3-di-n-butylaminopropoxy)benzoyl]-5-methylsulfonamidobenzofuran in the form of a pharmaceutically acceptable salt is selected from 2-n-butyl-3-[4-(3-di-n-butylaminopropoxy)benzoyl]-5-methylsulfonamidobenzofuran hydrochloride, 2-n-butyl-3-[4-(3-di-n-butylaminopropoxy)benzoyl]-5-methylsulfonamidobenzofuran fumarate and 2-n-butyl-3-[4-(3-di-n-butylaminopropoxy)benzoyl]-5-methylsulfonamidobenzofuran oxalate.

3. Composition according to either one of the preceding claims, **characterized in that** the active principle is selected from 2-n-butyl-3-[4-(3-di-n-butylaminopropoxy)benzoyl]-5-methylsulfonamidobenzofuran in the form of base and 2-n-butyl-3-[4-(3-di-n-butylaminopropoxy)benzoyl]-5-methylsulfonamidobenzofuran hydrochloride.

4. Composition according to any one of the preceding claims, **characterized in that** said composition further comprises at least one surfactant and/or at least one co-solvent.

5. Composition according to any one of the preceding claims, **characterized in that** said amphiphilic lipid excipient with HLB value between 5 and 18 has a melting point below 50°C.

6. Composition according to any one of the preceding claims, **characterized in that** it comprises 2-n-butyl-3-[4-(3-di-n-butylaminopropoxy)benzoyl]-5-methylsulfonamidobenzofuran hydrochloride, as active principle.

7. Composition according to any one of the preceding claims, **characterized in that** said amphiphilic lipid excipient with HLB value between 5 and 18 is selected from the lauroyl macroglyceride marketed under the brand name Gelucire® 44/14 and Gelucire® 50/13.

8. Composition according to any one of the preceding claims, **characterized in that** said amphiphilic lipid excipient with HLB value between 5 and 18 is the lauroyl macroglyceride marketed under the brand name Gelucire® 44/14.

9. Composition according to one of the preceding claims, **characterized in that** it comprises:
• 1-60 wt% of at least one active principle;
• 40-99 wt% of at least one lipid excipient;
• 0-30% of at least one compound selected from surfactants, co-solvents, diluents, disintegrants, lubricants, organic or inorganic bases and plasticizers,
the percentages being expressed by weight relative to the total weight of said composition.

10. Composition according to one of the preceding claims, **characterized in that** it comprises:
• 1-60 wt% of at least one active principle;
• 40-99 wt% of at least one lipid excipient,
• 0-30 wt% of at least one surfactant, and
• 0-29 wt% of at least one co-solvent;
the percentages being expressed by weight relative to the total weight of said composition.

11. Composition according to one of Claims 9 or 10, **characterized in that** it comprises:
• 1-50 wt% of at least one active principle, advantageously between 10 and 45%, even better between 20% and 40%;
and/or
• 45-80 wt% of at least one lipid excipient, advantageously between 50% and 60%;
and/or
• 1-20 wt% of at least one surfactant, advantageously between 5% and 15%;
and/or
• 1-20 wt% of at least one co-solvent, advantageously between 2 and 15%.

12. Composition according to one of Claims 9 to 11, **characterized in that** the surfactant is hydrophilic and nonionic.

13. Composition according to one of Claims 9 to 12, **characterized in that** the surfactant is selected from:
• ethylene oxide/propylene oxide copolymers;
• polyethoxylated castor oils;
• ethoxylated polysorbates, and
• polyethylene hydroxystearates.

14. Composition according to any one of Claims 9 to 13, **characterized in that** the surfactant is poloxamer 407.

15. Composition according to any one of Claims 9 to 14, **characterized in that** the co-solvent is selected from the alcoholic organic solvents or the glycol derivatives.

16. Dosage form comprising a composition according to any one of Claims 1 to 15.

17. Dosage form according to Claim 16, **characterized in that** it is a capsule selected from hard capsules, soft shell capsules, enteric capsules and modified-release capsules.

18. Dosage form according to either one of Claims 16 to 17, **characterized in that** it is a hard capsule.

19. Dosage form according to any one of Claims 16 to 18, **characterized in that** it contains between 50 and 500 mg of active principle, advantageously between 200 and 400 mg of active principle.
